# EUROPEAN PATENT SPECIFICATION

(11) **EP 4 313 954 B1**
(45) Date of publication and mention of the grant of the patent: **15.10.2025**
(21) Application number: 22715775.7
(22) Date of filing: 22.03.2022
(51) Int. Cl.: C07D 239/10, C07C 205/12, C07C 209/36, C07C 269/04, C07C 271/28, C07C 303/34, C07C 307/06, A01N 43/54

(54) **PREPARATION OF 2-CHLORO-4-FLUORO-5-NITROBENZOIC ACID**
HERSTELLUNG VON 2-CHLOR-4-FLUOR-5-NITROBENZOESÄURE
PRÉPARATION D'ACIDE 2-CHLORO-4-FLUORO-5-NITROBENZOÏQUE

(30) Priority: 26.03.2021 IN 202131013524
(43) Date of publication of application: 07.02.2024
(73) Proprietor: Adama Agan Ltd., 7710001 Ashdod (IL)
(72) Inventor: GRABARNICK, Michael, 8502500 Meitar (IL); JHA, Ashok Kumar, Hyderabad Telangana 500051 (IN); GALGE, Revanappa Vasantrao, Udgir, Latur Maharashtra 413517 (IN)
(74) Representative: Modiano, Gabriella Diana
(86) International application number: PCT/IL2022/050321
(87) International publication number: WO 2022/201155

(56) References cited:
- WO-A2-01/83459
- CN-B- 106 905 161
- BIN WANG ET AL: "New and convergent synthesis of saflufenacil", J. HETEROCYCLIC CHEM. 2020; 57, 1 January 2020 (2020-01-01), pages 151 - 156, XP055926461, Retrieved from the Internet <URL:https://onlinelibrary.wiley.com/doi/epdf/10.1002/jhet.3757> [retrieved on 20220531]

## Description

Saflufenacil (chemical name: 2-chloro-4-fluoro-5-[3-methyl-2,6-dioxo-4-(trifluoromethyl)-3,6-dihydropyrimidin-1(2H)-yl]-N-[methyl(propan-2-yl)sulfamoyl]benzamide) is an uracil (amide) herbicide. Saflufenacil is a pre-plant and pre-emergence herbicide applied alone or in combination with glyphosate to a wide range of food crops [see PPDB (Pesticide Properties Database created by the University of Hertfordshire https://sitem.herts.ac.uk/aeru/ppdb/en/index.htm)].

Saflufenacil was first described in WO 01/83459. The multistep synthesis of Saflufenacil includes the nitration of 2-chloro-4-fluorobenzoic acid to give 2-chloro-4-fluoro-5-nitrobenzoic acid. The reaction takes place in sulfuric acid, by addition of nitric acid, as reported in Example 1 of WO 01/83459. The reaction illustrated in WO 01/83459 shows 2-chloro-4-fluoro-5-nitrobenzoic acid as the sole reaction product:

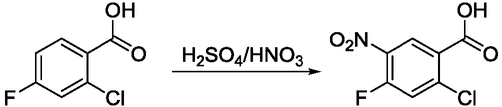

In fact, the reaction leads to a mixture of isomers, i.e., mononitration occurs at positions 5 or 3 of the aromatic ring:

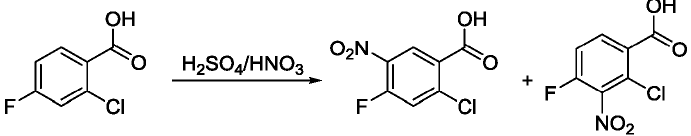

Hereinafter, 2-chloro-4-fluoro-5-nitrobenzoic acid and 2-chloro-4-fluoro-3-nitrobenzoic acid are referred to as the desired and undesired isomer, respectively. The amount of the undesired isomer is not insignificant, e.g., the crude reaction product usually consists of ~85-90:10-15 isomeric mixture.

Unfortunately, the undesired isomer is not easily separable from the crude product. Experimental results reported below indicate that purification of a ~85-90:10-15 isomeric mixture by crystallization to recover the desired isomer can only be achieved with an unacceptably large proportion of solvent and product. Multistep crystallizations were also met with difficulties.

We have found that the conditions of the mononitration reaction of 2-chloro-4-fluorobenzoic acid can be modified to advance a di-nitration side reaction, which occurs almost exclusively on the undesired isomer. That is, a di-nitro side product, namely, 3,5-dinitro-2-chloro-4-fluorobenzoic acid, is obtained at the expense of the undesired isomer. The undesired 3-nitro isomer undergoes consecutive aromatic nitration at position 5 rapidly, while the desired isomer does not react with the nitrating reagent. Hereinafter, the 3,5-dinitro-2-chloro-4-fluorobenzoic acid side product is named "3,5-dinitro side product".

The benefit gained from shifting the undesired isomer to double nitration is due to the increased solubility demonstrated by the resultant 3,5-dinitro side product in some types of organic solvents, compared to the isomers of the mononitration reaction. Consequently, the 3,5-dinitro side product can be easily separated from the crude product by crystallization, as opposed to the undesired isomer, which is difficult to remove. For example, a crude product consisting of ~ 85-90:3-9:9-3 proportioned mixture (HPLC, area %) of the desired isomer: undesired isomer: 3,5-dinitro side product can be purified by crystallization from, e.g., alkylated aromatic hydrocarbon solvent in an efficient manner, to recover the desired isomer in an essentially pure form. As pointed out above, the desired isomer is a useful intermediate, e.g., in the synthesis of herbicidally active compounds.

The invention is therefore primarily directed to a process, comprising reacting 2-chloro-4-fluorobenzoic acid with a nitrating agent;
isolating a crude reaction product consisting of 2-chloro-4-fluoro-5-nitrobenzoic acid, 2-chloro-4-fluoro-3-nitrobenzoic acid, and 3,5-dinitro-2-chloro-4-fluorobenzoic acid;
purifying the crude reaction product; and
obtaining 2-chloro-4-fluoro-5-nitrobenzoic acid in an essentially pure form.

The reaction is carried out under conditions advancing mononitration as a main reaction and di-nitration as a side reaction. A set of conditions enabling efficient management of the mononitration/di-nitration reactions include the nitrating agent, reaction temperature, and reaction time to adjust the ratio between the desired isomer, undesired isomer, and the di-nitro side product. A ratio (based on HPLC analysis, area %) of the undesired isomer and the 3,5-dinitro side product in the range of 3:1 to 1:3, e.g., 3:1 to 1:2, is usually satisfactory. A larger proportion of the di-nitro side product indicates that the reaction of the desired isomer with the nitrating agent occurred, leading to loss of yield. Thus, the abovementioned ratio strikes a balance between the benefit received from converting the undesired isomer to the easily separable side product and the loss of yield of the desired isomer. HPLC conditions are described in the experimental section below.

The nitration of 2-chloro-4-fluorobenzoic acid is preferably carried out with the nitric acid/sulfuric acid mixture. A reaction vessel is charged with concentrated sulfuric acid, followed by the addition of the starting material 2-chloro-4-fluorobenzoic acid, which readily dissolves in sulfuric acid at room temperature to afford a clear solution. The concentration of the starting material in the sulfuric acid is adjusted to provide a stirrable reaction mixture, usually not more than 25%. The addition of nitric acid to the mixture of sulfuric acid with the starting material is carried out gradually with cooling, keeping the temperature during the addition around room temperature (the addition is accompanied by the release of heat). Nitric acid can be supplied to the reaction in the form of 70% to 98% commercially available grades. A mixed acid reagent (H₂SO₄/HNO₃), prepared beforehand, e.g., usually as an equally proportioned mixture, can also be used. A molar excess of nitric acid is used to enable di-nitration to the extent required, namely at least 1.25 to 2.0 equivalents of nitric acid, preferably in the range of 1.4 to 1.8, e.g., around 1.5 equivalents (i.e., a molar excess of at least 25%, e.g., 25 to 100%, or 40% to 80% HNO₃ over the starting material). Upon completion of the addition of the nitric acid, the reaction mixture may be kept under stirring for the mononitration to reach completion at room temperature. Mononitration is usually carried out at a relatively low temperature, but in this case, because a di-nitration side reaction has been found to be advantageous, the reaction mixture is heated to a comparatively high temperature, at least above 55°C. The reaction temperature is usually in the range of 55 to 70°C, e.g., of 60 to 65°C or 65 to 70°C. While carrying out the nitration reaction of 2-chloro-4-fluorobenzoic acid at the temperature range set out above, the reaction time should be adjusted to get a distribution of, say, 80-90:3-10:3-10, e.g., of 85-90:3-9:3-9 (HPLC, area %) of the desired isomer, undesired isomer, and di-nitro product in the crude product, respectively. The reaction time is not less than 3 hours, e.g., not less than 4 hours, for example, from 4-7 hours (e.g., around 5 to 6 hours).

For example, one variant of the process comprises dissolving 2-chloro-4-fluorobenzoic acid in sulfuric acid, adding nitric acid at molar excess of at least 25% HNO₃, e.g., of 40% to 80% (around 50%), while cooling the reaction mixture, heating the reaction mixture to a temperature of not less than 55°C, and maintaining the reaction mixture at said temperature for at least 3 hours.

At the end of the reaction, the crude product is isolated by crystallization upon adding the reaction mixture to water at the temperature from 0 to 5°C, e.g., ice water, following which a solid is separated, e.g., by filtration or centrifugation, from the aqueous medium.

To this end, the contents of the reaction vessel are poured into ice water. The mixture is stirred well, for at least 30 minutes, e.g., for one to two hours, at the temperature of ~5-10 °C, whereby the solid crude product separates from the solution. It is better to use a comparably large volume of water, to minimize yield loss due to incomplete precipitation, as it appears that the product exhibits some solubility in the acidic aqueous medium, e.g., a volumetric ratio in the range of 2-3 to 30 (water/reaction mass). The solid that precipitated from the aqueous solution is recovered, e.g., by solid/liquid separation techniques such as filtration and centrifugation, and dried.

It appears that the crude product can pick up an appreciable amount of water during work-up. We were able to remove water in a reasonably efficient manner from the crude product by sucking on the filter for a few hours, reaching moisture levels of about 18 to 20% (Karl Fischer titration). Such moisture levels were found to be acceptable for the next step, i.e., recrystallization from an organic solvent. Other techniques of drying the crude product include vacuum drying in the oven or azeotropic distillation.

Next, the crude product (e.g., a 85-90:3-9:9-3 proportioned mixture) is purified by recrystallization from an organic solvent, i.e., a solvent, or a mixture of solvents, in which the di-nitro side product exhibits comparatively high solubility at room temperature or below, i.e., relative to the isomers of the mononitration reaction. A satisfactory difference in solubility was observed in low-medium polarity solvents (e.g., with a dielectric constant equal to or less than 20, for example, in the range from 2 to 20 at 20-25 °C, e.g., from 2 to 10), usually aprotic solvents, such as aromatic hydrocarbons (e.g., alkylated aromatic hydrocarbons, for example, toluene and xylene), halogenated aromatic solvents (for example, chlorobenzene), esters (for example, C2-C4 alkyl acetate) and ketones (e.g., methyl ethyl ketone). Purification by recrystallization is not limited to cooling crystallization or evaporation crystallization from a single solvent and includes also the use of solvent pairs. For example, from ethyl acetate/toluene 5-15/85-95 by volume solvent mixture. Aliphatic hydrocarbons, e.g., heptane, are usually inefficient when used alone but mixtures of an aliphatic hydrocarbon and a medium polarity solvent such as ethyl acetate or isopropanol can enable the isolation of a product with high purity levels. However, in general, higher recovery rates can be achieved by cooling crystallization from alkylated aromatic hydrocarbons, and this technique is generally preferred.

The proportion of an aromatic hydrocarbon solvent and crude product needed to achieve efficient recrystallization is in the range of 3/1 to 10/1, e.g., 4/1 to 9/1, for example, 5/1 to 8/1 (expressed as unit volume of solvent per unit weight of the dried crude product, e.g., ml/g). A typical crude product consists of ~80-90:3-10:3-10 (HPLC, area %) of the desired isomer, undesired isomer, and di-nitro side product, respectively. By efficient purification we mean achieving an industrially acceptable yield (>85%, e.g., >90%) and low amount of the undesired isomer impurity (<0.5%, e.g., <0.1%)]. The ratio between the solvent and the crude product is expected to vary depending on the type of solvent, targeted purity level and desired recovery rate, and will be adjusted accordingly. For example, toluene (5V to 7V) was shown to be an efficient solvent for recrystallization.

Drying of the recrystallized material can be carried out in vacuo at 45-50 °C for a few hours, to reach a moisture level of not more than 4%. Reducing the moisture level down to, say, 1% is not needed. 2-chloro-4-fluoro-5-nitrobenzoic acid appears to exhibit hygroscopicity to some extent, as it tends to absorb water and restore a water level of ~4%.

2-chloro-4-fluoro-5-nitrobenzoic acid is recovered in an essentially pure form, i.e., free of the undesired isomer, with purity level (by HPLC, area%) of not less than 97.0%, e.g., >98.0%, >99.0%, >99.5%.

As pointed out above, 2-chloro-4-fluoro-5-nitrobenzoic acid is useful as an intermediate in the synthesis of, inter alia, herbicidally active compounds such as saflufenacil depicted below:

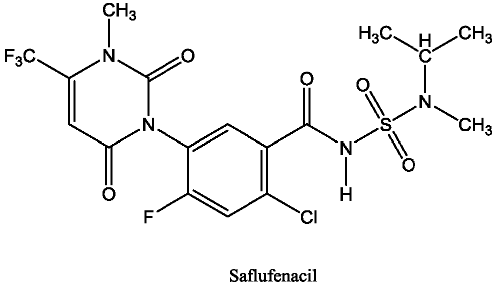

A process comprising converting the so-formed 2-chloro-4-fluoro-5-nitrobenzoic acid to the herbicidally active compound forms another aspect of the invention.

A few synthetic pathways can be employed to arrive at saflufenacil. For example, 2-chloro-4-fluoro-5-nitrobenzoic acid is reduced or hydrogenated to the corresponding amino compound 2-chloro-4-fluoro-5-aminobenzoic acid, e.g., with the aid of a metal reductant, such as iron or zinc, in an organic solvent, in the presence of an acid. For example, the reaction can take place in acetic acid as a solvent, using iron powder. 2-chloro-4-fluoro-5-aminobenzoic acid is then converted to the intermediate of Formula A1 depicted below, as shown in Examples 3 and 4 of WO 01/83459, through coupling with 2-dimethylamino-4-(trifluoromethyl)-6H, 1,3-oxazine-6-one in acetic acid, followed by alkylation in the presence of a base, e.g., to achieve methylation at the free nitrogen uracil ring and at the acid group (e.g., with methyl iodide and potassium carbonate, in a polar aprotic solvent such as dimethyl formamide)]:

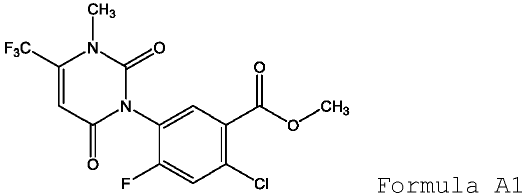

The ester intermediate of Formula A1 is cleaved to give the corresponding benzoic acid, for example, using BBr₃ as a deprotecting agent. The deprotection reaction takes place in, e.g., methylene chloride. The corresponding benzoic acid thus formed (chemically named 2-chloro-5-(3,6-dihydro-3-methyl-2,6-dioxo-4-(trifluoromethyl)-1(2H)-pyrimidinyl)-4-fluorobenzoic acid, CAS No. 120890-57-5) is collected in the form of a white solid (identified herein as compound of Formula A2):

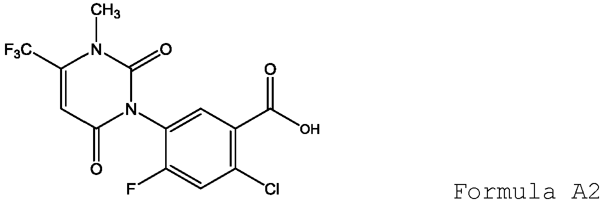

The acid reacts with NH₂-SO₂-N[(CH₃)(CH(CH₃)₂)], e.g., to afford saflufenacil. The acid can be activated, by conversion to acid chloride. More convenient, however, is to react the acid in the presence of N,N-carbonyldiimidazole (CDI) in tetrahydrofuran at reflux temperature, in the presence of diazabicycloundecene (DBU) .

Another important intermediate compound for the synthesis of Saflufenacil is the amino benzoylsulfamide of Formula B1:

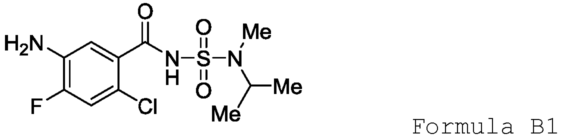

The preparation of the intermediate of Formula B1 includes condensation of 2-chloro-4-fluoro-5-nitrobenzoic acid with NH₂-SO₂-N[(CH₃)(CH(CH₃)₂)], namely, N-methyl-N-isopropylsulfamoyl amide (CAS No. 372136-76-0). The reaction can be carried out in the presence of N,N'-carbonyldiimidazole (CDI) and diazabicycloundecene (DBU) in tetrahydrofuran (THF) as a solvent, as shown in Example 54 of WO 01/83459 to give the corresponding nitro benzoylsulfamide. Reduction of the nitro group as in the previously described route of synthesis, in, e.g., acetic acid and THF as cosolvents using iron powder, gives the amino benzoylsulfamide of Formula B1. Also possible is the hydrogenation of the nitro group with Raney nickel in methanol (see hydrogenation procedure in Example 31 of US 7,820,846). Preparation of the intermediate B1 from the acid is illustrated by the reaction scheme depicted below:

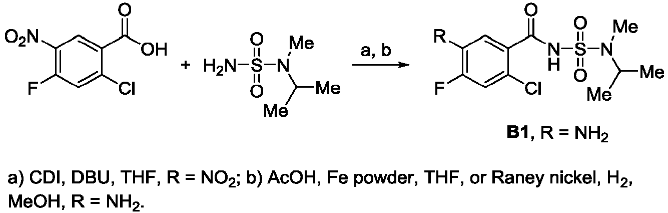

Saflufenacil is accessible from the intermediate of Formula B1 through various routes.

For example, based on WO 01/83459, the coupling of the intermediate of Formula B1 with 2-dimethylamino-4-(trifluoromethyl)-6H, 1,3-oxazine-6-one in acetic acid gave des-methyl saflufenacil of Formula B2:

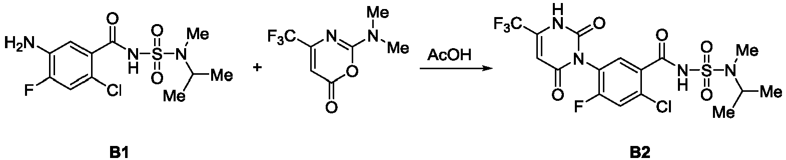

Other approaches involve a cyclization step to form the uracil ring system.

One synthetic route is based on the conversion of the amine group of the intermediate of Formula B1 to isocyanate and coupling it with an ethyl 3-amino-4,4,4-trifluoro-2-butenoate. The preparation of the phenyl isocyanate is described in US 7,820,846. The amino benzoylsulfamide of Formula B1 was treated with a phosgenating agent to afford the corresponding phenyl isocyanate. Preparation of saflufenacil precursor of Formula B2 (namely, des-methyl saflufenacil) from the isocyanate by coupling it with enamine is described in US 7,737,275. The synthetic route is shown below:

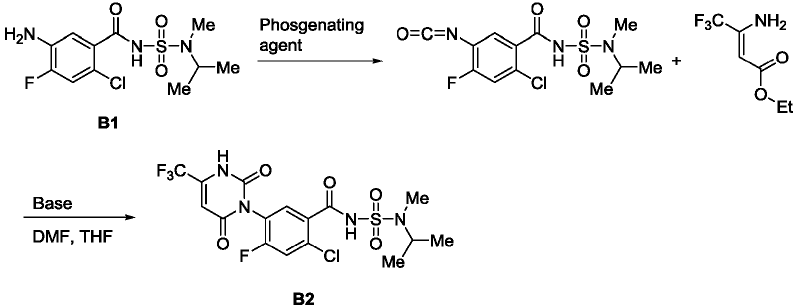

The coupling of the phenyl isocyanate with enamine is performed under an inert atmosphere (water is removed from the reaction vessel by azeotrope drying before the addition of the isocyanatobenzoyl). Different types of bases can be used, e.g., sodium hydride, potassium methoxide, and potassium tert-butoxide as well as potassium carbonate, e.g., MeOK in Example 2 of US 7,737,275.

Another synthetic pathway involving a cyclization step to form the uracil ring system is found in US 8,252,925. The amino benzoylsulfamide intermediate of Formula B1 is treated with a solution of ethyl chloroformate (e.g., in methylene chloride in the presence of pyridine) to give the corresponding ethylate (see Example 1.2 of US 8,252,925). Ring closure is accomplished by reacting the ethylate with ethyl 3-amino-4,4,4-trifluoro-2-butenoate (e.g., in DMF) to form the uracil ring (see Example 3.1.a of US 8,252,925). Resulting des-methyl saflufenacil of Formula B2 is alkylated with dimethyl sulfate (e.g. in toluene/THF) in the presence of an aqueous base, with phase transfer catalyst to afford the targeted active ingredient (see Example 3.3 of US 8,252,925). An exemplary synthetic pathway is depicted below:

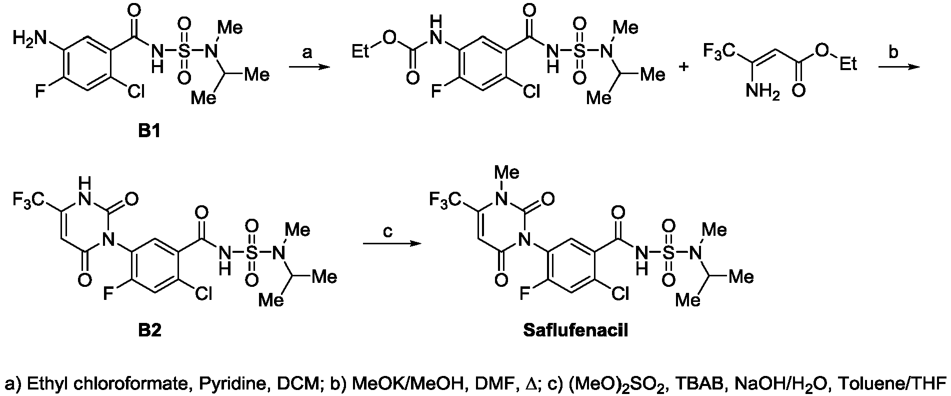

Accordingly, 2-chloro-4-fluoro-5-nitrobenzoic acid prepared according to the invention can be used to produce saflufenacil by processes comprising the following steps:
a) condensing the 2-chloro-4-fluoro-5-nitrobenzoic acid with N-methyl-N-isopropylsulfamoyl amide to give nitro benzoylsulfamide, followed by reduction or hydrogenation of the nitro group to form the amine compound of Formula B1; 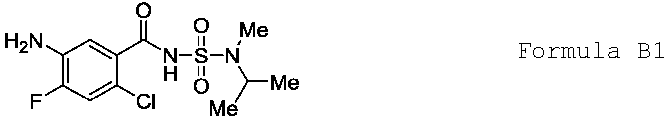
b) preparing a compound of Formula B2 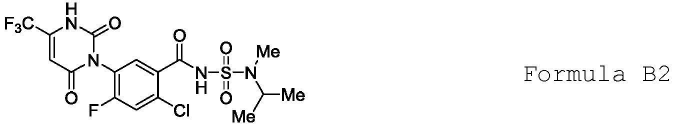 from the compound of Formula B1 by:
   b1) coupling the compound of Formula B1 with 2-dimethylamino-4-(trifluoromethyl)-6H, 1,3-oxazine-6-one; or
   b2) converting the compound of Formula B1, e.g., with the aid of a phosgenating agent, to isocyanate, and reacting the isocyanate with enamine; or
   b3) reacting the compound of Formula B1 with ethyl chloroformate, followed by coupling with an enamine; and
c) methylation of the compound of Formula B2 to afford saflufenacil.

Another way to synthesize saflufenacil consists of the next steps:
a) esterification of 2-chloro-4-fluoro-5-nitrobenzoic acid with an alcohol, in the presence of, e.g., Thionyl chloride, to give alkyl 2-chloro-4-fluoro-5-nitrobenzoate: 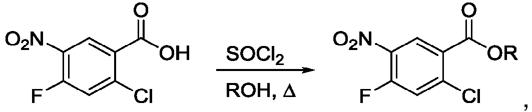 wherein R is selected from a group consisting of C₁₋₁₂ straight or branched alkyl (e.g., C₁₋₅) which may be substituted with 1 or more substituents, a C₃₋₁₀ cycloalkyl, which may be substituted with 1 or more substituents, a C₆₋₁₀ aromatic ring which may be substituted with 1 or more substituents, a C₅₋₁₀ heteroaromatic ring, which may be substituted with 1 or more substituents;
b) reduction or hydrogenation of the said alkyl 2-chloro-4-fluoro-5-nitrobenzoate to form alkyl 2-chloro-4-fluoro-5-aminobenzoate. The reduction or hydrogenation reaction can be performed in a suitable reactor, e.g., high pressure reactor, autoclave, etc., with a conventional catalyst such as Pd on activated carbon, Raney nickel, etc., using different solvents, preferably aprotic, e.g., isopropyl acetate, THF, DMF, etc.: 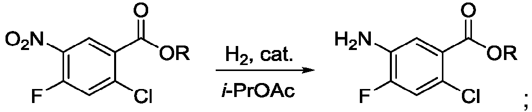
c) transforming alkyl 2-chloro-4-fluoro-5-aminobenzoate to carbamate alkyl 2-chloro-4-fluoro-5-aminobenzoate. For example, the reaction of alkyl 2-chloro-4-fluoro-5-aminobenzoate with ethyl chloroformate in the presence of *N,N-*diethylaniline gives the desired carbamate: 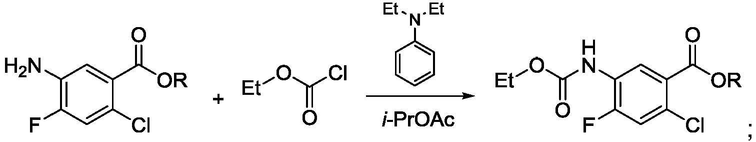
d) cyclization of carbamate alkyl 2-chloro-4-fluoro-5-aminobenzoate to obtain alkyl 2-chloro-5-(2,6-dioxo-4-(trifluoromethyl)-3,6-dihydropyrimidin-1(2H)-yl)-4-fluorobenzoate. The cyclization is performed using 1,8-diazabicyclo(5.4.0)undec-7-ene (DBU) and ethyl 3-amino-4,4,4-trifluorobut-2-enoate: 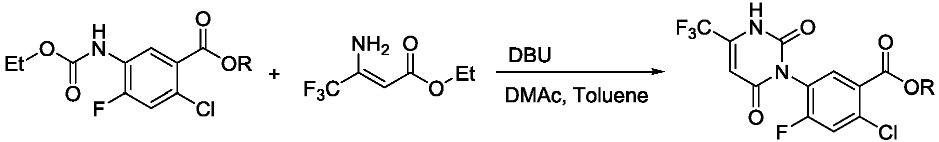
e) hydrolysis of alkyl 2-chloro-5-(2,6-dioxo-4-(trifluoromethyl)-3,6-dihydropyrimidin-1(2H)-yl)-4-fluorobenzoate to form 2-Chloro-5-(2,6-dioxo-4-(trifluoromethyl)-3,6-dihydropyrimidin-1(2*H*)-yl)-4-fluorobenzoic acid. The hydrolysis can be performed in usual conditions known from the art, e.g., in a protic or aprotic solvent at acid/base conditions, to give the acid of Formula A3: 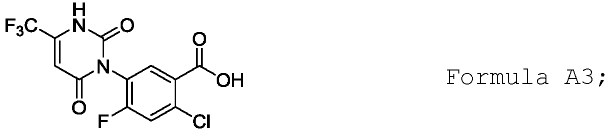
f) coupling said benzoic acid with NH₂-SO₂-N[(CH₃)(CH(CH₃)₂)] to afford the compound of Formula B2: 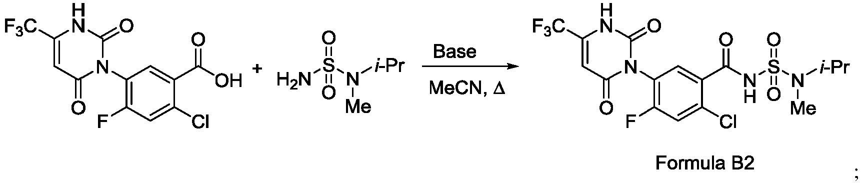
g) methylation of the compound of Formula B2 to afford saflufenacil.

### Examples

HPLC method conditions: Column: XBridge C18 (4.6x150)mm, 3.5µm; Mobile phase-A: 10 ammonium bicarbonate in water; Mobile Phase - B: acetonitrile; Gradient (T/%B): 0/10, 10/90, 15/90, 16/10, 20/10; Flow rate: 1 mL/min; Diluent: acetonitrile/water (1:1 v/v); injection volume: 5µL; run time: 20 minutes). [RRT of undesired isomer 1.22, RRT of dinitro 1.45].

### Example 1

### Preparation and purification of 2-chloro-4-fluoro-5-nitrobenzoic acid

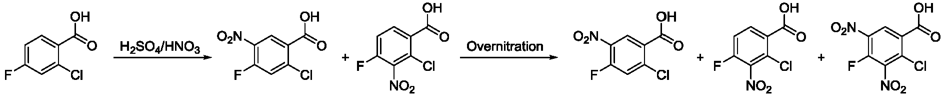

A 1-L four-necked flask equipped with a mechanical stirrer, dropping funnel, reflux condenser, and thermocouple was charged with concentrated sulfuric acid (500 mL) and 2-Chloro-4-fluorobenzoic acid (100 g, 0.57 mol) at ambient temperature. The mixture was stirred for 10-15 min to obtain a clear solution and cooled to 10-15 °C. Concentrated HNO₃ (54 g, 0.86 mol, 1.5 eq.) was added dropwise and the obtained heterogeneous mixture was heated to 60-70 °C. The homogenous reaction mixture formed was stirred for 5-6 h at 60-70 °C with HPLC monitoring [HPLC protocol consisted of 1) quenching 1 ml sample taken from the reaction mixture in 1 ml of ice cold water, shaking for one minute, adding HPLC diluent (acetonitrile/water (1:1) mixture to obtain a clear solution, and HPLC analysis to detect < 8% of the undesired isomer and progressive formation of the dinitro byproduct]. On completion of the reaction, the reaction mass is carefully poured into 2 L of ice-cold water for 30-40 min at below 5-10 °C. Brown colored fumes were observed during quenching. The reaction mass was stirred at 5-10 °C for another 1-2 h (during which time solid precipitation was completed), centrifuged, and spin-dried during 1 - 2 hours.

The solid was added to Toluene (500 mL) and the mixture was heated to 75-80 °C and stirred for 2-4 h. The obtained clear solution was slowly cooled to room temperature for 4-5 h. A precipitate was formed during the cooling. The precipitate was isolated by filtration under vacuum for 1-2 h. The filtrate was collected for recovery and the filter cake was dried in vacuo at 45-50 °C for 8 h to obtain the desired isomer (89.0 g, 71.2%) with 99.6% purity by HPLC.

### Examples 2 (comparative) and 3-5 (invention)

### Preparation of 2-chloro-4-fluoro-5-nitrobenzoic acid: the effect of the nitrating agent

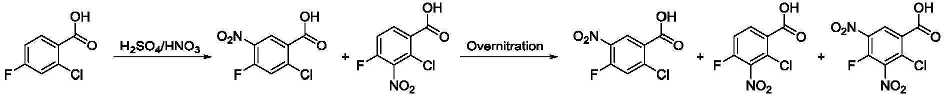

In a series of experiments, the procedure of Example 1 was repeated, carrying out the reaction at 55-60/60-70 °C for three hours under varying amounts of the nitrating agent. The composition of the crude product (desired isomer, undesired isomer, and the 3,5-dinitro side product) was determined by HPLC analysis and the results are tabulated below.

**Table 1**

| Example | HNO₃(eq) | 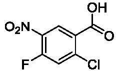 HPLC (area %) | 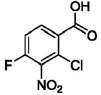 HPLC (area %) | 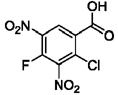 HPLC (area %) |
|---|---|---|---|---|
| 2 (comparative) | 1 | 83.60 | 11.52 | ND* |
| 3 (invention) | 1.25 | 81.08 | 12.30 | 2.80 |
| 4 (invention) | 1.5 | 88.22 | 3.38 | 5.8 |
| 5 (invention) | 2.0 | 71.49 | 3.53 | 14.01 |
| * Not detected | | | | |

### Examples 6 7 and 8 (invention)

### Preparation of 2-chloro-4-fluoro-5-nitrobenzoic acid: the effect of reaction temperature

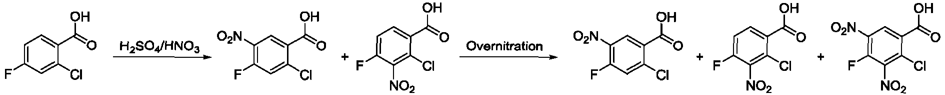

In a series of experiments, the procedure of Example 1 was repeated, varying the reaction temperature: 55-60 °C, reaction carried out for four hours (Example 6); 60-65 °C, reaction carried out for three hours (Example 7); and 70 to 75 °C, reaction carried out for four hours (Example 8). The composition of the crude product (desired isomer, undesired isomer and the 3,5-dinitro side product) was determined by HPLC analysis and the results are tabulated below.

**Table 2**

| Example | T(°C) | t(h) | 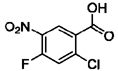 HPLC (area %) | 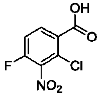 HPLC (area %) | 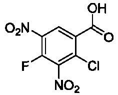 HPLC (area %)) |
|---|---|---|---|---|---|
| 6 (invention) | 55-60 | 4 | 75.53 | 10.99 | 8.12 |
| 7 (invention) | 60-65 | 3 | 85.10 | 8.74 | 3.51 |
| 8 (invention) | 70-75 | 4 | 56.5 | 33.71 | 5.95 |

### Examples 9 (comparative) and 10 to 12 (of the invention)

### Preparation of 2-chloro-4-fluoro-5-nitrobenzoic acid: the effect of reaction time

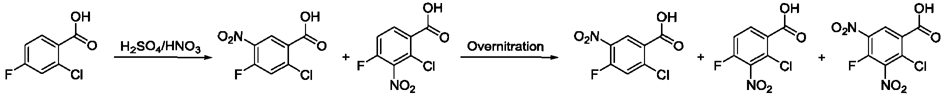

In a series of experiments, the procedure of Example 1 was repeated, varying the duration of the reaction, which was continued for two hours (Example 9); three hours (Example 10), four hours (Example 11), and 5 hours (Example 12). The composition of the crude product (consisting of the desired isomer, undesired isomer and the 3,5-dinitro side product) was determined by HPLC analysis and the results are tabulated below.

**Table 3**

| Example | T (°C) | t(h) | 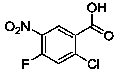 HPLC (area %) | 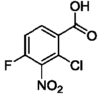 HPLC (area %) | 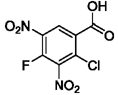 HPLC (area %) |
|---|---|---|---|---|---|
| 9 (comparative) | 65-70 | 2 | 82.88 | 10.03 | 4.59 |
| 10 (invention) | 65-70 | 3 | 81.09 | 8.14 | 7.14 |
| 11 (invention) | 65-70 | 4 | 79.90 | 8.25 | 8.53 |
| 12 (invention) | 65-70 | 5 | 77.71 | 7.44 | 11.06 |

### Examples 13A-13D (comparative)

### Preparation and purification of 2-chloro-4-fluoro-5-nitrobenzoic acid: mononitration without di-nitration side reaction

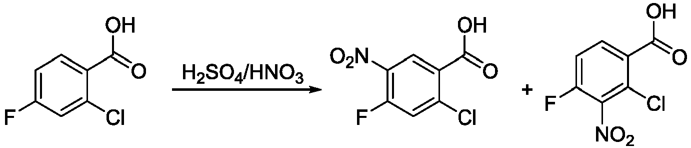

A 1-L four-necked flask equipped with a mechanical stirrer, dropping funnel, reflux condenser, and thermocouple was charged with concentrated sulfuric acid (500 mL) and 2-Chloro-4-fluorobenzoic acid (100 g, 0.57 mol) at ambient temperature. The mixture was stirred for 10-15 min to obtain a clear solution and cooled to 0-5 °C. Concentrated HNO₃ (43g, 0.69 mol, 1.2 eq.) was added dropwise and the mixture was allowed to warm and stirred for 1-1.5 h at room temperature with HPLC monitoring.

After finishing, the reaction mixture was added 1 L of ice water. The resulting suspension was stirred at 0-5 °C for 1 h, allowed to warm to ambient temperature, and filtered. The cake was washed with 200 mL of water and dried in vacuo. The solid consisted of 86.35% and 12.22% of the desired and undesired isomers, respectively, with no detectable amounts of the 3,5-dinitro side product.

A series of experiments was carried out to purify typical crude products obtained by the procedure set out above, by recrystallization from a solvent pair (isopropyl alcohol/n-heptane). Experimental data (crude weight, the proportion by volume of the solvents in the solvent mixture, and recrystallization conditions) are tabulated below.

**Table 4**

| Ex. | Crude (g) | Solvent pair | Crystallization conditions |
|---|---|---|---|
| 13A | 36 g | IPA(1.5V)/n-heptane(15V) | Crude was charged to IPA; n-heptane was added dropwise under stirring at 0-5°C; the mixture was maintained for 60 min; solid was isolated by filtration. |
| 13B | 5 g | IPA(0.75)/ n-heptane (14.25V) | Crude was charged to IPA/n-heptane mixture and maintained for one hour at 60-70 °C; the solution was cooled to 10 °C; the mixture was maintained for 30 min; the solid was isolated by filtration. |
| 13C | 5 g | IPA(2.0)/ n-heptane (10.0V) | Crude was charged to IPA; n-heptane is added dropwise under stirring at 20 °C; the mixture was maintained for 60 min; solid was isolated by filtration. |
| 13D | 10 g | IPA(2.0)/ n-heptane (10.0V) | Crude was charged to IPA; n-heptane was added dropwise under stirring at 10-15 °C; the mixture was maintained for 60 min; the solid was isolated by filtration. |

A crude product consisting of the isomeric mixture of the mononitration reaction, lacking the di-nitro derivative, was difficult to purify by recrystallization from the isopropyl alcohol/n-heptane system. For example, under the conditions of Examples 13A and 13B, reasonable recovery was observed but product purity was unacceptable (94% and 87%, respectively). Recrystallization according to Examples 13C and 13D afforded highly pure product, but recovery percentage was poor (<20% and <40%, respectively), and attempts to collect a second crop product resulted in a significant drop of purity levels.

### Examples 14A-14F (saflufenacil synthesis)

### Preparation of saflufenacil using 2-chloro-4-fluoro-5-nitrobenzoic acid as starting material

### Example 14A. Ethyl 2-chloro-4-fluoro-5-nitrobenzoate.

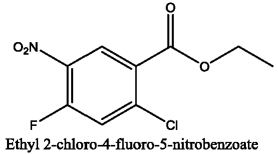

To a clean, dry 4 necks RBF (2000 mL) equipped with a mechanical stirrer, thermocouple, condenser and additional funnel were charged 500 mL ethanol at 25-30 °C and 100g of 2-chloro-4-fluoro-5-nitrobenzoic acid, and the mixture was stirred for 10-15 mins up to get a clear solution. The reaction mass was cooled to 0-5 °C and 108.6g of Thionyl chloride (2.0 eq.) was fed to the reaction mass at 0-5 °C for about 30 mins. After that, the reaction mass was slowly heated to 65-70 °C and stirred at this temperature with mild reflux of solvent during 6-8 hrs at 65-70 °C. The reaction was monitored by HPLC area % analysis up to a residual concentration of 2-chloro-4-fluoro-5-nitrobenzoic acid less than 1%. After the reaction was finished, about 400 mL of ethanol were distilled out at 60-65 °C under reduced pressure. The reaction mass was cooled to 20-25 °C and 500 mL of water were added to the reaction mass over the period of 15-20 mins at 20-25 °C. After that, 500 mL of isopropyl acetate were added at once to the reaction mass and the mixture was stirred for 15-20 mins. The layers were separated at 25-30 °C. The top isopropyl acetate layer contains the product. A solution in isopropyl acetate was washed with 100 mL of 2% sodium bicarbonate aqueous solution. After phases separation, isopropyl acetate solution of ethyl 2-chloro-4-fluoro-5-nitrobenzoate may be delivered to the next step (hydrogenation of the nitro-group) without additional purification and/or product separation. The yield of ethyl 2-chloro-4-fluoro-5-nitrobenzoate is 98%.

### Example 14B. Ethyl 5-amino-2-chloro-4-fluorobenzoate.

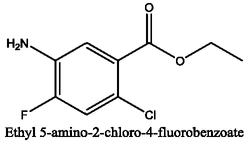

Isopropyl acetate solution of ethyl 2-chloro-4-fluoro-5-nitrobenzoate from Example 14A was introduced to the clean and dry pressure reactor equipped with a mechanical stirrer, manometer and thermocouple at 25-30 °C. To the solution 15g of Raney Ni were added under nitrogen atmosphere at the same temperature. The reactor was closed, and hydrogen gas pressure was applied to 90-100 PSI at 25-30 °C. The mixture was stirred at the same pressure of hydrogen for 26-30 hrs at 25-30 °C up to the concentration both of starting material and hydroxylamine intermediate were reduced below 1 area % according to HPLC. At the end of the reaction, the catalyst was filtered from reaction mass through celite bed under nitrogen atmosphere at 25-30 °C. The bed was washed with 100 mL of isopropyl acetate at 25-30 °C. To the combined Isopropyl acetate solution were added 200 mL of Isopropyl acetate and the same volume of the solvent was distilled out at 80-85 °C under atmospheric pressure to dry the reaction mixture up to the moisture content level 0.5% by KF analysis. The reaction mass was cooled to 25-30 °C and analyzed. Ethyl 5-amino-2-chloro-4-fluorobenzoate in isopropyl acetate solution may be delivered to the next step (carbamate preparation) without additional purification and/or product separation. The yield of ethyl 5-amino-2-chloro-4-fluorobenzoate is 95%.

### Example 14C. Ethyl 2-chloro-5-ethoxycarbonylamino-4-fluorobenzoate.

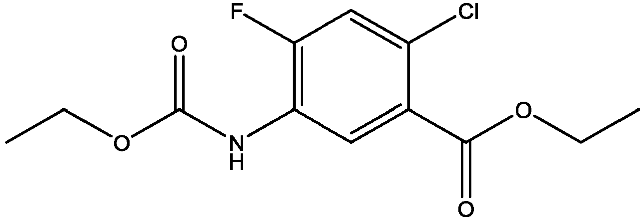

### Ethyl 2-chloro-5-ethoxycarbonylamino-4-fluorobenzoate

To the solution of ethyl 5-amino-2-chloro-4-fluorobenzoate in isopropyl acetate from Example 14B 102.5g of N,N-diethylaniline were added at 25-30 °C. To this mixture 74g of ethyl chloroformate were fed dropwise over the period of 15-20 mins at 25-30 °C. The reaction mass was heated to 40-45 °C and maintained at this temperature for 6-8 hrs up to the reduction of starting material concentration below 1 area % by HPLC. Towards the end of the reaction, solid precipitation was observed. The reaction mass was cooled to 25-30 °C and 300 mL of 10% HCl were added at this temperature. The reaction mass was stirred at 25-30 °C for 30-40 mins and after that two layers were separated. An aqueous layer was sent for recovery of N,N-diethylaniline. The upper organic layer was washed with 100 mL of 5% aqueous sodium bicarbonate solution and analyzed. Ethyl 2-chloro-5-ethoxycarbonylamino-4-fluorobenzoate in isopropyl acetate solution may be delivered to the next step (cyclization) without additional purification and/or product separation. The yield of ethyl 2-chloro-5-ethoxycarbonylamino-4-fluorobenzoate is 97%.

### Example 14D. Ethyl 2-chloro-5-(2,6-dioxo-4-(trifluoromethyl)-3,6-dihydropyrimidin-1(2H)-yl)-4-fluorobenzoate.

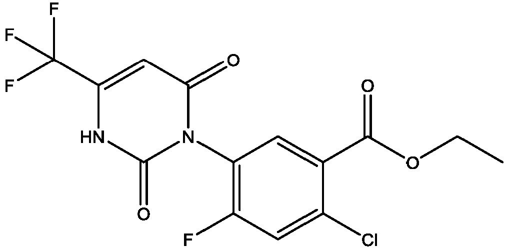

### Ethyl 2-chloro-5-(2,6-dioxo-4-(trifluoromethyl)-3,6-dihydropyrimidin-1(2H)-yl)-4-fluorobenzoate

Isopropyl acetate solution of ethyl 2-chloro-5-ethoxycarbonylamino-4-fluorobenzoate from Example 14C was charged into clean and dry RBF and most of the isopropyl acetate (about 300-350 mL) was distilled out at 55-60 °C at 600-625 mbar. To the residue were added 300 mL of *N,N-*dimethylacetamide, and distillation of isopropyl acetate was continued at the same conditions. Toward the end of distillation to the mixture were added 200 mL of toluene and the mixture was dried by azeotropic distillation of the toluene at 60-70 °C under the vacuum. Residual water content must be not more than 0.5% by KF. To the dry solution of ethyl 2-chloro-5-ethoxycarbonylamino-4-fluorobenzoate in *N,N-*dimethylacetamide 104.4 g of 1,8-diazabicyclo(5.4.0)undec-7-ene (DBU) and 100.8 g of ethyl 3-amino-4,4,4-trifluorobut-2-enoate were added at 25-30 °C. The reaction mass was heated to 58-62 °C under a nitrogen stream for better removal of ethanol formed in the reaction. The reaction mass was stirred at these conditions for 12-14 hrs, so that concentration of ethyl 2-chloro-5-ethoxycarbonylamino-4-fluorobenzoate was reduced below 2 area % by HPLC. The reaction mass was cooled to 25-30 °C and poured to 500 mL of 10% aqueous HCl at the temperature 10-15 °C. The temperature was raised 4-5 °C and with stirring the reaction mass was warmed to 25-30 °C. To the mixture 1000 mL of isopropyl acetate were added and stirring continued for 30-40 mins at 25-30 °C. The layers were separated at 25-30 °C. Isopropyl acetate contains the product. Ethyl 2-chloro-5-(2,6-dioxo-4-(trifluoromethyl)-3,6-dihydropyrimidin-1(2*H*)-yl)-4-fluorobenzoate in isopropyl acetate solution may be delivered to the next step (hydrolysis) without additional purification and/or product separation. The yield of ethyl 2-chloro-5-(2,6-dioxo-4-(trifluoromethyl)-3,6-dihydropyrimidin-1(2*H*)-yl)-4-fluorobenzoate is 85%.

### Example 14E. 2-Chloro-5-(2,6-dioxo-4-(trifluoromethyl)-3,6-dihydropyrimidin-1(2H)-yl)-4-fluorobenzoic acid.

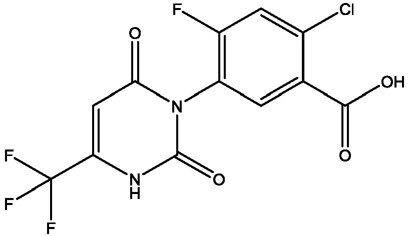

### 2-Chloro-5-(2,6-dioxo-4-(trifluoromethyl)-3,6-dihydropyrimidin-1(2H)-yl)-4-fluorobenzoic acid

Ethyl 2-chloro-5-(2,6-dioxo-4-(trifluoromethyl)-3,6-dihydropyrimidin-1(2*H*)-yl)-4-fluorobenzoate in isopropyl acetate solution from Example 14D was delivered into cleaned RBF at 25-30 °C and about 850 mL of the isopropyl acetate were distilled out at 45-50 °C under reduced pressure. To the mixture 600 mL of Dioxane were added and about 100 mL of Dioxane together with the rest of isopropyl acetate were distilled out under vacuum at 55-60 °C. To the reaction mass 1000 mL of concentrated HCl were slowly fed at 25-30 °C. The reaction mass was heated to 90-95 °C and stirred at this condition for 22-24 hrs up to reduction of ethyl 2-chloro-5-(2,6-dioxo-4-(trifluoromethyl)-3,6-dihydropyrimidin-1(2*H*)-yl)-4-fluorobenzoate concentration below 2 area % by HPLC. At the end of the reaction, Dioxane was distilled out at 55-60°C under reduced pressure. The reaction mass was cooled to 25-30°C and 700 mL of water were added at the same temperature. Stirring was continued for 4-6 hrs at 20-25 °C. The reaction product was filtered at 25-30 °C and washed with 400 mL of water at 25-30 °C. 2-Chloro-5-(2,6-dioxo-4-(trifluoromethyl)-3,6-dihydropyrimidin-1(2*H*)-yl)-4-fluorobenzoic acid was prepared with the purity not less than 96 area % by HPLC. The wet compound was dispersed in 500 mL of Toluene and the mixture was dried by azeotropic distillation at 100-110 °C up to moisture content in the reaction mass not more than 0.5% by KF. The reaction mass was cooled to 20-25 °C and stirred at this temperature during 3-4 hrs. 2-Chloro-5-(2,6-dioxo-4-(trifluoromethyl)-3,6-dihydropyrimidin-1(2*H*)-yl)-4-fluorobenzoic acid was filtered at 20-25 °C and dried under reduced pressure at 45-50 °C. 108g of the product was separated with assay 97%. The yield is 86% on the hydrolysis step or 66% on five telescopic steps.

### Example 14F. 2-Chloro-5-(2,6-dioxo-4-(trifluoromethyl)-3,6-dihydropyrimidin-1(2H)-yl)-4-fluoro-N-(N-isopropyl-N-methylsulfamoyl)benzamide.

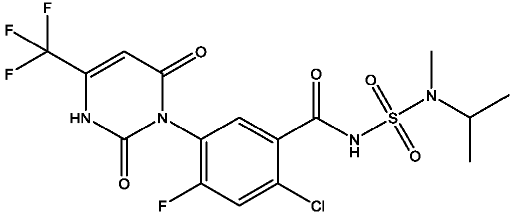

### 2-Chloro-5-(2,6-dioxo-4-(trifluoromethyl)-3,6-dihydropyrimidin-1(2H)-yl)-4-fluoro-N-(N-isopropyl-N-methylsulfamoyl)benzamide

To a clean, dry 4 necks RBF equipped with a mechanical stirrer, thermocouple, condenser and additional funnel were charged 40 ml of acetonitrile, 13g of *N*-isopropyl*-N*-methylsulfamoylamide and 15.75g of potassium carbonate at 25-30 °C and under nitrogen atmosphere. The reaction mass was heated to attain 55-60 °C.

Into another RBF were charged 100 mL of acetonitrile and under nitrogen atmosphere was added 20g of 2-chloro-5-(2,6-dioxo-4-(trifluoromethyl)-3,6-dihydropyrimidin-1(2*H*)-yl)-4-fluorobenzoic acid from Example 14E and 12g of 1,1'-carbonyldiimidizole (CDI) at 25-30 °C. The reaction mass was heated to 55-60 °C and stirred at this temperature during about 1.5h to produce 3-(4-chloro-2-fluoro-5-(1*H*-imidazole-1-carbonyl)phenyl)-6-(trifluoromethyl)pyrimidine-2,4(1*H*,3*H*)-dione. Residual concentration of 2-chloro-5-(2,6-dioxo-4-(trifluoromethyl)-3,6-dihydropyrimidin-1(2*H*)-yl)-4-fluorobenzoic acid was below 2 area % by HPLC.

A prepared solution of 3-(4-chloro-2-fluoro-5-(1*H*-imidazole-1-carbonyl)phenyl)-6-(trifluoromethyl)pyrimidine-2,4(1*H*,3*H*)-dione was fed to the mixture of *N*-isopropyl-*N*-methylsulfamoylamide and potassium carbonate over the period of 10-15 mins at 55-60 °C. The reaction mass was stirred at 55-60 °C for 6-8 hrs up to the moment that concentration of 3-(4-chloro-2-fluoro-5-(1*H-*imidazole-1-carbonyl)phenyl)-6-(trifluoromethyl)pyrimidine-2,4(1*H*,3*H*)-dione was not more than 2 area % by HPLC. At the end of the reaction, the mixture was cooled to 25-30 °C and stirred at this temperature for 25-30 mins. K₂CO₃ was filtered from the reaction mass at 25-30 °C and washed with 20 mL of acetonitrile. The filtrate (containing the product) was charged into clean RBF and heated to 40-45 °C. About 80 mL of acetonitrile was distilled from the filtrate at 40-45 °C under reduced pressure (650 mbar). The reaction mass was cooled to 25-30 °C and 200 mL of 2-methyl-THF and 100 mL of water were added at once. With good stirring, the reaction mass was cooled to 0-5 °C and the pH of the reaction mass was adjusted to 1-2 with concentrated HCl (about 25 mL) at the same temperature. Cooling and stirring were stopped, and layers were separated at 25-30 °C. The top organic layer contained the product. The bottom aqueous layer contained imidazole hydrochloride. The top organic layer was charged to the clean RBF and 60 mL of water were added at 25-30 °C. With good stirring, the pH of the aqueous phase was adjusted to 5.8-6.0 with 5% aqueous sodium bicarbonate. The layers were separated at 25-30 °C. The top organic layer contained the product. The bottom aqueous layer contained sodium salt of 2-chloro-5-(2,6-dioxo-4-(trifluoromethyl)-3,6-dihydropyrimidin-1(2*H*)-yl)-4-fluorobenzoic acid. The top organic layer was charged into clean RBF and about 160 mL of 2-methyl THF were distilled out at 40-45 °C under reduced pressure. To the residue, 60 mL of toluene were added, and an additional about 30 mL of the solvent (mainly the rest of 2-methyl THF) were distilled out at the same conditions. The reaction mass was cooled and stirred for 1-2 hrs at 25-30 °C. Precipitated solid was filtered and washed with 20 ml of toluene at 25-30 °C. Wet solid was charged into clean RBF and 40 ml of acetone were added at 25-30 °C. The mixture was stirred for 30 mins at 25-30 °C and 100 mL of water were fed slowly at the same temperature. The mixture was stirred during 4 hrs for full crystallization of the product and the solid compound was filtered at 25-30 °C. After drying at 50-55 °C under reduced pressure for 10-12 hrs, 19g of 2-chloro-5-(2,6-dioxo-4-(trifluoromethyl)-3,6-dihydropyrimidin-1(2*H*)-yl)-4-fluoro-*N*-(*N*-isopropyl-*N*-methylsulfamoyl)benzamide were prepared. Yield 70%.

The next step (methylation, to afford Saflufenacil) may be carried on by using prior art examples, for instance of the WO 2001/083459 publication.

## Claims

1. A process comprising the steps of:
reacting 2-chloro-4-fluorobenzoic acid with a nitrating agent;
isolating a crude reaction product consisting of 2-chloro-4-fluoro-5-nitrobenzoic acid, 2-chloro-4-fluoro-3-nitrobenzoic acid and 3,5-dinitro-2-chloro-4-fluorobenzoic acid;
purifying the crude reaction product; and
recovering 2-chloro-4-fluoro-5-nitrobenzoic acid in an essentially pure form;
wherein the nitrating agent is a nitric acid/sulfuric acid mixture, with a molar excess of at least 25% HNO₃ relative to 2-chloro-4-fluorobenzoic acid;
the process comprising dissolving 2-chloro-4-fluorobenzoic acid in sulfuric acid, adding nitric acid while keeping the reaction mixture cool, heating the reaction mixture to a temperature of not less than 55°C, and maintaining the reaction mixture at said temperature for at least 3 hours.

2. A process according to claim 1, wherein the crude product is isolated by crystallization upon adding the reaction mixture to cold water, following which the crude solid product is separated from the aqueous medium.

3. A process according to claim 2, wherein the isolated crude product is 85-90:3-9:9-3 proportioned mixture (HPLC, area %).

4. A process according to any one of claims 1 to 3, wherein the crude product is purified by recrystallization from one or more solvents.

5. A process according to claim 4, wherein the solvent(s) is (are) selected from the group consisting of aromatic hydrocarbon solvents, halogenated aromatic solvents, ester solvents and ketone solvents.

6. A process according to claim 5, wherein the solvent is alkylated aromatic hydrocarbon.

7. A process according to claim 6, wherein the proportion of the aromatic hydrocarbon solvent and the crude product is in the range of 4/1 to 9/1.

8. A process according to any one of the preceding claims, further comprising the steps of:
reducing or hydrogenating 2-chloro-4-fluoro-5-nitrobenzoic acid to give 2-chloro-4-fluoro-5-aminobenzoic acid;
transforming 2-chloro-4-fluoro-5-aminobenzoic acid into an ester compound of Formula A1: 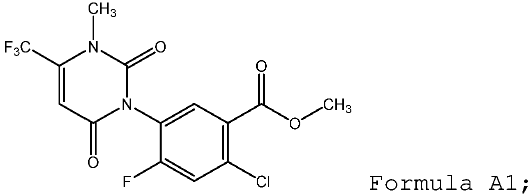
cleaving said ester of Formula A1 to the corresponding benzoic acid of Formula A2: 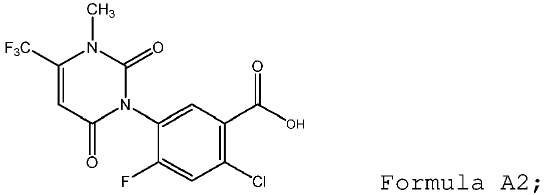 and
reacting said benzoic acid A2 with NH₂-SO₂-N[(CH₃)(CH(CH₃)₂)] to afford saflufenacil;
or
comprising the steps of:
a) condensing the 2-chloro-4-fluoro-5-nitrobenzoic acid with N-methyl-N-isopropylsulfamoyl amide to give nitro benzoylsulfamide, followed by reduction or hydrogenation of the nitro group to form the amine compound of Formula B1; 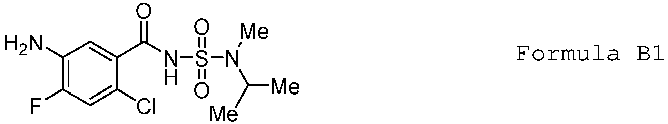
b) preparing a compound of Formula B2 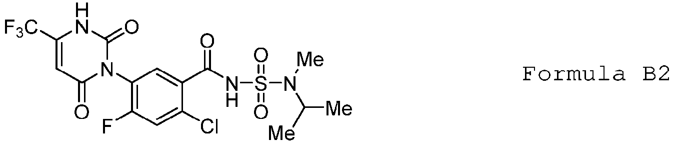 from the compound of Formula B1 by:
b1) coupling the compound of Formula B1 with 2-dimethylamino-4-(trifluoromethyl)-6H, 1,3-oxazine-6-one; or
b2) converting the compound of Formula B1 to the corresponding isocyanate, and reacting the isocyanate with enamine; or
b3) reacting the compound of Formula B1 with ethyl chloroformate, followed by coupling with an enamine; and
c) methylation of the compound of Formula B2 to afford saflufenacil;
or
comprising the steps of:
a) esterification of 2-chloro-4-fluoro-5-nitrobenzoic acid with an alcohol to give alkyl 2-chloro-4-fluoro-5-nitrobenzoate: 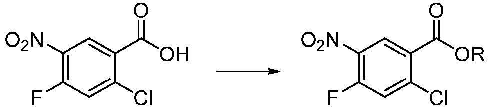 wherein R is a straight or branched C1-C5 alkyl;
b) reduction or hydrogenation of alkyl 2-chloro-4-fluoro-5-nitrobenzoate to form alkyl 2-chloro-4-fluoro-5-aminobenzoate: 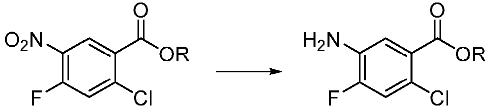
c) transforming alkyl 2-chloro-4-fluoro-5-aminobenzoate to carbamate alkyl 2-chloro-4-fluoro-5-aminobenzoate: 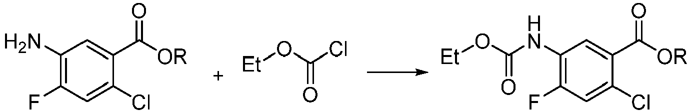
d) cyclization of carbamate alkyl 2-chloro-4-fluoro-5-aminobenzoate to obtain alkyl 2-chloro-5-(2,6-dioxo-4-(trifluoromethyl)-3,6-dihydropyrimidin-1(2H)-yl)-4-fluorobenzoate: 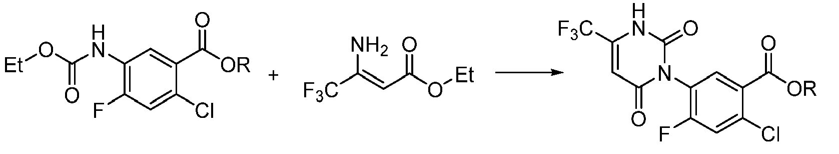
e) hydrolysis of alkyl 2-chloro-5-(2,6-dioxo-4-(trifluoromethyl)-3,6-dihydropyrimidin-1(2H)-yl)-4-fluorobenzoate to form 2-Chloro-5-(2,6-dioxo-4-(trifluoromethyl)-3,6-dihydropyrimidin-1(2*H*)-yl)-4-fluorobenzoic acid of formula A3: 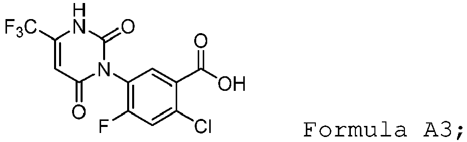
f) reaction of said benzoic acid of Formula A3 with NH₂-SO₂-N[(CH₃)(CH(CH₃)₂)] to give a compound of Formula B2: 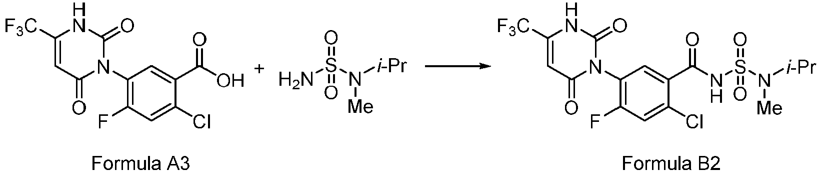 and
g) methylation of the compound of Formula B2 to afford saflufenacil.

## Patentansprüche

1. Ein Verfahren, das folgende Schritte umfasst:
das Reagieren von 2-Chlor-4-fluorbenzoesäure mit einem Nitrierungsmittel;
das Isolieren eines rohen Reaktionsprodukts, das aus 2-Chlor-4-fluor-5-nitrobenzoesäure, 2-Chlor-4-fluor-3-nitrobenzoesäure und 3,5-Dinitro-2-chlor-4-fluorbenzoesäure besteht;
das Reinigen des rohen Reaktionsprodukts; und
das Rückgewinnen von 2-Chlor-4-fluor-5-nitrobenzoesäure in einer im Wesentlichen reiner Form;
wobei das Nitrierungsmittel eine Mischung aus Salpetersäure und Schwefelsäure mit einem molaren Überschuss von mindestens 25% HNO₃ relativ zu 2-Chlor-4-fluorbenzoesäure ist;
wobei das Verfahren Folgendes umfasst: das Auflösen von 2-Chlor-4-fluorbenzoesäure in Schwefelsäure, das Hinzufügen von Salpetersäure bei gleichzeitigem Kühlhalten der Reaktionsmischung, das Erhitzen der Reaktionsmischung auf eine Temperatur nicht niedriger als 55°C und das Halten der Reaktionsmischung bei der Temperatur über mindestens 3 Stunden.

2. Ein Verfahren gemäß Anspruch 1, worin das Rohprodukt isoliert wird durch Kristallisation nach Hinzugeben der Reaktionsmischung zu kaltem Wasser, wonach das feste Rohprodukt vom wässerigen Medium getrennt wird.

3. Ein Verfahren gemäß Anspruch 2, worin das isolierte Rohprodukt eine Mischung im Verhältnis 85-90:3-9:9-3 ist (HPLC, Bereich %).

4. Ein Verfahren gemäß einem beliebigen der Ansprüche 1 bis 3, worin das Rohprodukt durch Umkristallisieren aus einem oder mehreren Lösungsmitteln gereinigt wird.

5. Ein Verfahren gemäß Anspruch 4, worin das/die Lösungsmittel gewählt ist/sind aus der Gruppe bestehend aus aromatischen Kohlenwasserstoff-Lösungsmitteln, halogenierten aromatischen Lösungsmitteln, Ester-Lösungsmitteln und Keton-Lösungsmitteln.

6. Ein Verfahren gemäß Anspruch 5, worin das Lösungsmittel alkylierter aromatischer Kohlenwasserstoff ist.

7. Ein Verfahren gemäß Anspruch 6, worin das Verhältnis des aromatischen Kohlenwasserstoff-Lösungsmittels und des Rohprodukts im Bereich von 4/1 bis 9/1 liegt.

8. Ein Verfahren gemäß einem beliebigen der obigen Ansprüche, das weiter folgende Schritte umfasst:
das Reduzieren oder Hydrieren von 2-Chlor-4-fluor-5-nitrobenzoesäure, um 2-Chlor-4-fluor-5-aminobenzoesäure zu ergeben;
das Umwandeln von 2-Chlor-4-fluor-5-aminobenzoesäure in eine Esterverbindung mit der Formel A1: 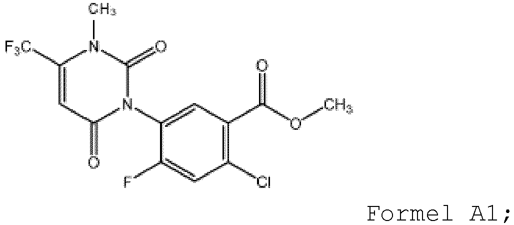
das Aufspalten des Esters mit der Formel A1 in die entsprechende Benzoesäure mit der Formel A2: 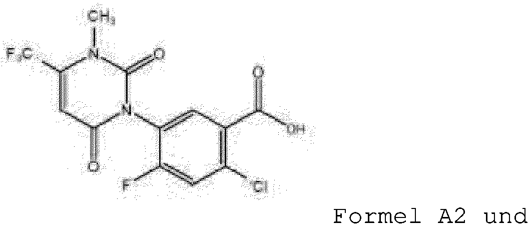
das Reagieren der Benzoesäure A2 mit NH₂-SO₂-N[(CH₃)(CH(CH₃)₂)], um Saflufenacil zu ergeben;
oder
das folgende Schritte umfasst:
a) Kondensieren der 2-Chlor-4-fluor-5-nitrobenzoesäure mit N-Methyl-N-isopropylsulfamoylamid, um Nitrobenzoylsulamid zu ergeben, gefolgt von Reduktion oder Hydrierung der Nitrogruppe, um die Aminverbindung mit der Formel B1 zu bilden; 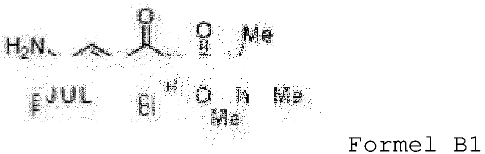
b) Herstellen einer Verbindung mit der Formel B2 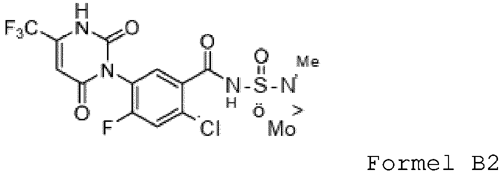 aus der Verbindung mit der Formel B1 durch:
b1) Kopplung der Verbindung mit der Formel B1 mit 2-Dimethylamino-4-(trifluormethyl)-6H, 1,3-oxazin-6-on; oder
b2) Umwandlung der Verbindung mit der Formel B1 in das entsprechende Isocyanat und Reagieren des Isocyanats mit Enamin; oder
b3) Reagieren der Verbindung mit der Formel B1 mit Ethylchlorformiat, gefolgt von Kopplung mit einem Enamin; und
c) Methylierung der Verbindung mit der Formel B2, um Saflufenacil zu ergeben;
oder
das folgende Schritte umfasst:
a) Veresterung von 2-Chlor-4-fluor-5-nitrobenzoesäure mit einem Alkohol, um Alkyl-2-chlor-4-fluor-5-nitrobenzoat zu ergeben: 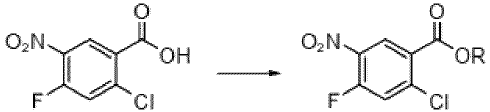 worin R ein gerades oder verzweigtes C₁-C₅-Alkyl ist;
b) Reduktion oder Hydrierung von Alkyl-2-chlor-4-fluor-5-nitrobenzoat, um Alkyl-2-chlor-4-fluor-5-aminobenzoat zu bilden; 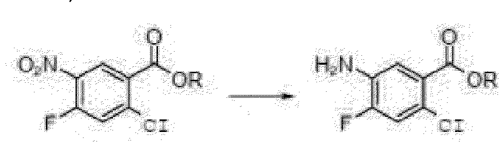
c) Umwandeln von Alkyl-2-chlor-4-fluor-5-aminobenzoat in Carbamat-alkyl-2-chlor-4-fluor-5-aminobenzoat: 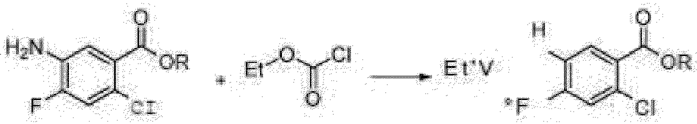
d) Cyclisierung von Carbamat-alkyl-2-chlor-4-fluor-5-aminobenzoat, um Alkyl-2-chlor-5-(2,6-dioxo-4-(trifluormethyl)-3,6-dihydropyrimidin-1(2H)-yl)-4-fluorbenzoat zu erhalten: 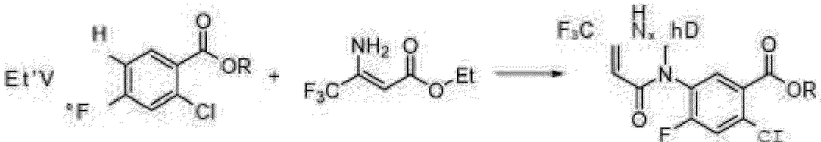
e) Hydrolyse von Alkyl-2-chlor-5-(2,6-dioxo-4-(trifluormethyl)-3,6-dihydropyrimidin-1(2H)-yl)-4-fluorbenzoat, um 2-Chlor-5-(2,6-dioxo-4-(trifluormethyl)-3,6-dihydropyrimidin-1(2H)-yl)-4-fluorbenzoesäure mit der Formel A3 zu bilden: 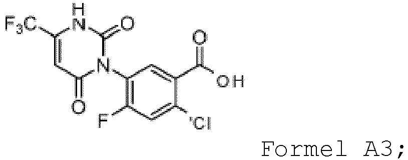
f) Reaktion der Benzoesäure mit der Formel A3 mit NH₂-SO₂-N[(CH₃)(CH(CH₃)₂)], um eine Verbindung mit der Formel B2 zu ergeben: 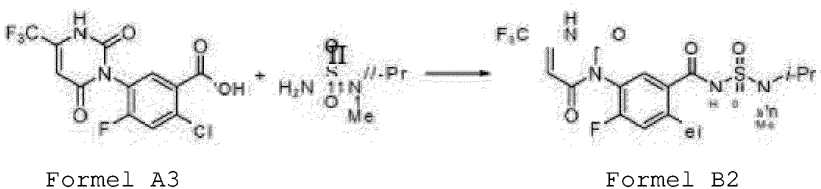 und
g) Methylierung der Verbindung mit der Formel B2, um Saflufenacil zu ergeben.

## Revendications

1. Procédé comprenant les étapes suivantes :
faire réagir de l'acide 2-chloro-4-fluorobenzoïque avec un agent nitrant ;
isoler un produit de réaction brut constitué d'acide 2-chloro-4-fluoro-5-nitrobenzoïque, d'acide 2-chloro-4-fluoro-3-nitrobenzoïque et d'acide 3,5-dinitro-2-chloro-4-fluorobenzoïque ;
purifier le produit de réaction brut ; et
récupérer l'acide 2-chloro-4-fluoro-5-nitrobenzoïque sous une forme essentiellement pure ;
où l'agent nitrant est un mélange d'acide nitrique et d'acide sulfurique, avec un excès molaire d'au moins 25 % de HNO₃ par rapport à l'acide 2-chloro-4-fluorobenzoïque ;
le procédé comprenant la dissolution de l'acide 2-chloro-4-fluorobenzoïque dans l'acide sulfurique, l'ajout d'acide nitrique tout en maintenant le mélange réactionnel à une température basse, le chauffage du mélange réactionnel à une température d'au moins 55 °C et le maintien du mélange réactionnel à ladite température pendant au moins 3 heures.

2. Procédé selon la revendication 1, dans lequel le produit brut est isolé par cristallisation après addition du mélange réactionnel à de l'eau froide, après quoi le produit solide brut est séparé du milieu aqueux.

3. Procédé selon la revendication 2, où le produit brut isolé est un mélange dans les proportions 85-90:3-9:9-3 (HPLC, % de surface).

4. Procédé selon l'une quelconque des revendications 1 à 3, où le produit brut est purifié par recristallisation à partir d'un ou plusieurs solvants.

5. Procédé selon la revendication 4, dans lequel le ou les solvant(s) est (sont) choisis dans le groupe constitué par les solvants hydrocarbonés aromatiques, les solvants aromatiques halogénés, les solvants esters et les solvants cétones.

6. Procédé selon la revendication 5, où le solvant est hydrocarbure aromatique alkylé.

7. Procédé selon la revendication 6, où la proportion du solvant hydrocarboné aromatique et du produit brut est comprise entre 4/1 et 9/1.

8. Procédé selon l'une quelconque des revendications précédentes, comprenant en outre les étapes suivantes :
réduire ou hydrogéner l'acide 2-chloro-4-fluoro-5-nitrobenzoïque pour donner l'acide 2-chloro-4-fluoro-5-aminobenzoïque ;
transformer l'acide 2-chloro-4-fluoro-5-aminobenzoïque en un composé ester de formule Al : 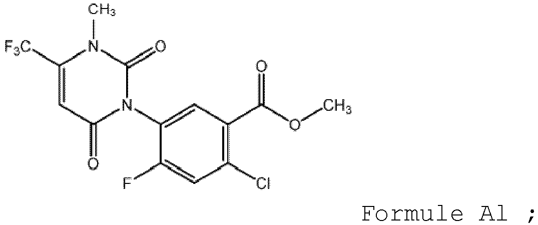
cliver ledit ester de formule Al en l'acide benzoïque correspondant de formule A 2 : 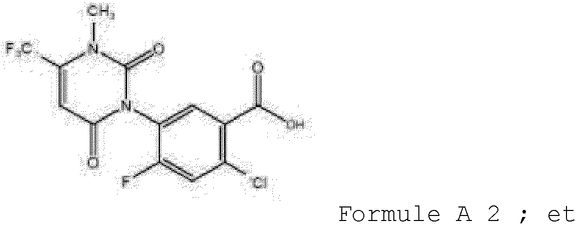
faire réagir ledit acide benzoïque A2 avec NH₂-SO₂-N[(CH₃)(CH(CH₃)₂)] pour obtenir le saflufenacil ;
ou
comprenant les étapes suivantes :
a) condensation de l'acide 2-chloro-4-fluoro-5-nitrobenzoïque avec le N-méthyl-N-isopropylsulfamoylamide pour donner le nitro benzoylsulfamide, suivie d'une réduction ou d'une hydrogénation du groupe nitro pour former le composé aminé de formule B1 ; 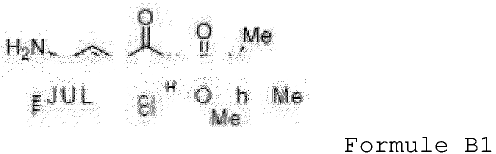
b) préparer un composé de formule B2 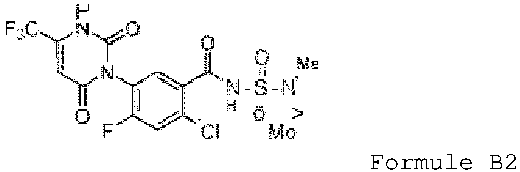 à partir du composé de formule Bl par :
bl) coupler le composé de formule B1 avec 2-diméthylamino-4-(trifluorométhyl)-6H, 1,3-oxazine-6-one ; ou
b2) convertir le composé de formule B1 en l'isocyanate correspondant, et faire réagir l'isocyanate avec une énamine ;
ou
b3) faire réagir le composé de formule B1 avec du chloroformiate d'éthyle, suivie d'un couplage avec une énamine ; et
c) la méthylation du composé de formule B2 pour obtenir le saflufenacil ;
ou
comprenant les étapes suivantes :
a) estérification de l'acide 2-chloro-4-fluoro-5-nitrobenzoïque avec un alcool pour donner le 2-chloro-4-fluoro-5-nitrobenzoate d'alkyle : 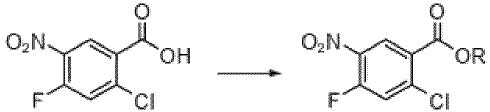 où R est un groupe alkyle en C₁-C₅ linéaire ou ramifié ;
b) réduction ou hydrogénation de l'alkyl 2-chloro-4-fluoro-5-nitrobenzoate pour former l'alkyl 2-chloro-4-fluoro-5-aminobenzoate : 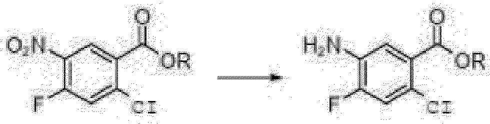
c) transformer l'alkyl 2-chloro-4-fluoro-5-aminobenzoate en carbamate d'alkyl 2-chloro-4-fluoro-5-aminobenzoate : 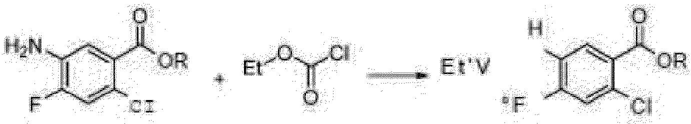
d) cyclisation du carbamate d'alkyle 2-chloro-4-fluoro-5-aminobenzoate pour obtenir l'alkyle 2-chloro-5-(2,6-dioxo-4-(trifluorométhyl)-3,6-dihydropyrimidin-1(2H)-yl)-4-fluorobenzoate : 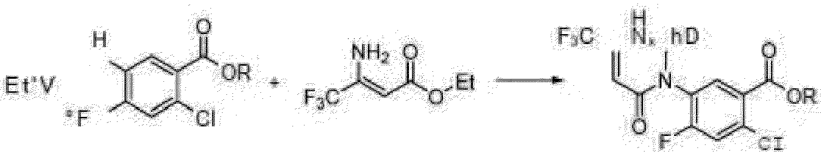
e) hydrolyse de l'alkyl 2-chloro-5-(2,6-dioxo-4-(trifluorométhyl)-3,6-dihydropyrimidin-1(2H)-yl)-4-fluorobenzoate pour former de l'acide 2-chloro-5-(2,6-dioxo-4-(trifluorométhyl)-3,6-dihydropyrimidin-1(2H)-yl)-4-fluorobenzoïque de formule A 3 : 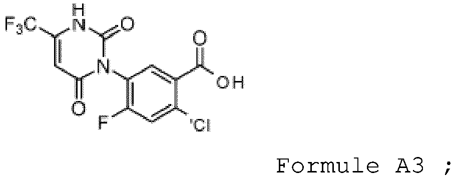
f) réaction dudit acide benzoïque de formule A3 avec NH₂-SO₂-N[(CH₃)(CH(CH₃)₂)] pour donner un composé de formule B2 : 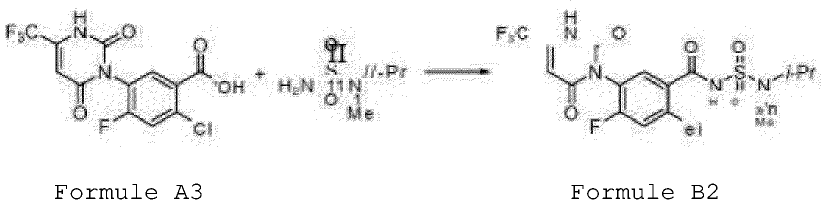 et
g) méthylation du composé de formule B2 pour obtenir le saflufenacil.
